# EUROPEAN PATENT APPLICATION

(11) **EP 1 955 997 A1**
(43) Date of publication of application: **13.08.2008**
(21) Application number: 08002481.3
(22) Date of filing: 11.02.2008
(51) Int. Cl.: C07C 253/30, C07C 255/25, C07C 209/48, C07C 211/09

(54) **Selective manufacture of N, N'-bis(cyanoethyl)-1,2-ethylenediamine and N,N'-bis(3-aminopropyl)-1,2-ethylenediamine**

(30) Priority: 12.02.2007 US 673697
(71) Applicant: Air Products and Chemicals, Inc., Allentown, PA 18195-1501 (US)
(72) Inventor: Hayes, Kathryn Sue, Plymouth Meeting, PA 19462 (US); Vedage, Gamini Ananda, Bethlehem, PA 18017 (US); Lutz, Eugene George, Lenhartsville, PA 19534 (US); Barrall, Zane W., Hamburg, PA 19526 (US)
(74) Representative: Kador & Partner

(57) **Abstract**

A method for making N,N'-bis(3-aminopropyl)-1,2-ethylenediamine which comprises reacting acrylonitrile and ethylenediamine in about a 2:1 molar ratio to make a N,N'-bis(cyanoethyl)-1,2-ethylenediamine reaction product and hydrogenating the reaction product, the improvement for improving the selectivity of the reactions to N,N'-bis(2-cyanoethyl)-ethylenediamine and to N,N'-bis(3-aminopropyl)-1,2-ethylenediamine which comprises reacting acrylonitrile and ethylenediamine in the presence of 2-30 wt% water, based on total reactants.

## Description

### BACKGROUND OF THE INVENTION

N,N'-bis(3-aminopropyl)ethylenediamine is produced in two steps: cyanoethylation of ethylenediamine (EDA) followed by hydrogenation of the cyanoethylated product to the corresponding amine. Under anhydrous conditions and when acrylonitrile is added to EDA at a molar ratio of 2:1, the cyanoethylation reaction produces a mixture of mono-, di-, and tricyanoethylated products.

DE 2 446 489 discloses acrylonitrile was added over 2 hours to EDA in a 2:1 molar ratio containing acetic acid. The product was distilled allegedly to give a 98% yield of N,N'-bis(cyanoethyl)ethylenediamine. However, the present inventors believe the analytical method used to determine the yield of N,N'-bis(2-cyanoethyl)ethylenediamine was not capable of distinguishing this compound from a mixture containing the mono-, di- and tricyanoethylated ethylenediamines. Moreover, the corrosiveness of acetic acid strongly discourages the use of this process commercially.

L. G. Duquette, et al, Ind. Eng. Chem. Prod. Res. Dev., 21, 632-635, 1982, disclose cyanoethylation of ethylenediamine to afford a statistical distribution of products based on mole ratio. Product composition (mono:di) was only influenced by mole ratio; being independent of residence time, temperature, or catalysts (acetic acid).

DE 2 739 917 discloses polyalkylene polyamines prepared by adding acrylonitrile (1 mol) slowly to EDA (1 mol). The product was hydrogenated in the presence of NH₃ to give 70% N-(2-aminoethyl)-1,3-propanediamine and 20% N,N'-ethylenebis(1,3-propanediamine).

US 4 094 802 discloses N,N,N',N'-tetrakis(2-cyanoethyl)ethylenediamine was prepared by addition of 6 moles acrylonitrile to 1 mole ethylenediamine in 5.5 moles water (water concentration = 21 wt%) at 25-40 °C. The product was hydrogenated in ethanol solvent over Raney nickel catalyst.

GB 2 067 191 discloses preparation of a polyamine by hydrogenating a polynitrile in the presence of a pelleted Co-Zn catalyst. Polynitrile substrates were prepared by reaction of EDA with acrylonitrile.

US 5 434 262 discloses in Example 1 the preparation of N,N'-bis(3-aminopropyl)-ethylenediamine by adding 3 moles of acrylonitrile to 1 mole of EDA over 3 hours. The product was isolated in 60% yield. Upon hydrogenation of the dinitrile (10 g) in ethanol over Raney catalyst afforded N,N'-bis(3-aminopropyl)ethylenediamine in 60% yield.

US 5 750 788 dislcoses preparation of N,N,N',N'-tetrakis(cyanoethyl)-1-2-ethylene-diamine by addition of 5 moles acrylonitrile to 1 mole ethylenediamine in water as a solvent (water concentration was about 68 wt%) over 90 min. The product was dissolved in N-methylpyrrolidone (NMP) and hydrogenated in a continuous reactor over Co/Mn/P catalyst in the presence of ammonia.

K. M. Taylor et al, J. Am. Chem. Soc. 1959, 81, 5333-5335, discloses that water is a catalyst for the cyanoethylation of t-carbinamines.

US 6 245 932 discloses the cyanoethylation of substituted cycloaliphatic vicinal diamines which comprises reacting acrylonitrile and a vicinal diamine in the presence of water as a catalyst.

### BRIEF SUMMARY OF THE INVENTION

The present invention is directed to the selective manufacture of N,N'-bis(2-cyanoethyl)-1,2-ethylenediamine and N,N'-bis(3-aminopropyl)-1,2-ethylenediamine.

In an embodiment there is disclosed a method for making N,N'-bis(2-cyanoethyl)-1,2-ethylenediamine which comprises reacting acrylonitrile and ethylenediamine in a 1.6-2.4:1 molar ratio in the presence of 2-30 wt% water, based on total reactants.

According to the present invention, addition of 2-30 wt% water to the reaction mixture dramatically increases the selectivity to the desired dicyanoethylated product yielding a mono-, bis- and tris- product distribution wt% ratio of 1 to 10 : 80 to 95 : 1 to 10, respectively.

As a further embodiment of the invention hydrogenation of the nitrile mixture under semi-batch conditions over a hydrogenation catalyst provides the desired N,N'-bis(3-aminopropyl)ethylenediamine in >80 wt%, preferably >85 wt%, yield.

### DETAILED DESCRIPTION OF THE INVENTION

A method is provided for preferentially making N,N'-bis(2-cyanoethyl)-1,2-ethylenediamine which comprises reacting acrylonitrile and ethylenediamine in the presence of 2-30 wt% water, based on total reactants, in semi-batch mode, i.e., 1.6 to 2.4 moles acrylonitrile added to one mole EDA over a period of time.

There is also provided a method for selectively making N,N'-bis(3-aminopropyl)-1,2-ethylenediamine which comprises reacting acrylonitrile and ethylenediamine in the presence of 2-30 wt% water, based on total reactants, to make a N,N'-bis(2-cyanoethyl)-1,2-ethylenediamine-containing reaction mixture and hydrogenating the reaction mixture in a semi-batch reaction over a hydrogenation catalyst.

In one embodiment the acrylonitrile is added to the ethylenediamine over a period of time, i.e., a semi-batch reaction. In another embodiment the acrylonitrile and the ethylenediamine are both added continuously and simultaneously as separate streams or as a mixture to the reaction. The water may be added mixed with either of the reactants or added separately.

In each above embodiment the acrylonitrile and ethylenediamine are reacted in a molar ratio of about 1.6 - 2.4 to 1, i.e., 1.6 - 2.4 moles acrylonitrile per mole ethylenediamine, preferably 1.8 - 2.2 to 1, most preferably about 2 to 1, at a temperature from 20 to 90 °C, preferably 40 to 70 °C and atmospheric or autogenous pressure in the presence of 2 to 30 wt% water, preferably 3 to 25 wt%, and especially 5 to 20 wt% water, based on combined weight of water, acrylonitrile and ethylenediamine, to afford a (cyanoethyl)ethylene-diamines product mixture that is at least 80 wt% N,N'-bis(2-cyanoethyl)ethylenediamine, preferably at least 85 wt%.

The (cyanoethyl)ethylenediamines product mixture is hydrogenated in a semi-batch reaction to give a (3-aminopropyl)ethylenediamines product mixture that is at least 80 wt% N,N'-bis(3-aminopropyl)ethylenediamine, preferably at least 85 wt%. The hydrogenation reaction utilizes, as is well known in the art, hydrogen pressures of 6 to 110 bar, preferably 20 to 90 bar, especially 30 to 70 bar and temperatures from 70 to 150 °C, preferably 100 to 130 °C. Suitable hydrogenation catalysts for use in the reaction are those typically used and well known in the art of hydrogenating nitriles to amines, in particular the Raney metal catalysts, for example, Raney cobalt 2724 catalyst from Grace's Davison division.

Batch reaction means the reactants are mixed together and allowed to react until the reactants are completely converted to product. Semi-batch reaction means one reactant (in this case acrylonitrile) is added to the other reactant (ethylenediamine), ideally at least at the rate at which the reaction occurs; i.e., the reactants are converted essentially to product by the time the addition is done. If the addition rate is faster than the reaction rate, additional reaction time will be needed at the end of the addition to complete the reaction.

In the following Examples the following abbreviations were used:
EDA = ethylenediamine
ACN = acrylonitrile
CNEDA = N-2-cyanoethyl-1,2-ethylenediamine
BCNEDA = N,N'-bis(2-cyanoethyl)-1,2-ethylenediamine
TCNEDA = N,N,N'-tris(2-cyanoethyl)-1,2-ethylenediamine
AEPD = N-(2-aminoethyl)-1,3-propanediamine = N-3-aminopropyl-1,2-ethylenediamine
BAPED = N,N'-bis(3-aminopropyl)ethylenediamine
TAPED = N,N,N'-tris(3-aminopropyl)ethylenediamine

### Cyanoethylation of Ethylenediamine (EDA)

### Example 1

A 100 ml glass reactor equipped with mechanical stirrer, condenser, nitrogen inlet, and feed inlet was charged with 18.4 g (0.31 mol) EDA. The reactor was purged with nitrogen for a few minutes then the agitator was started, and the reactor was heated to 50 °C. When the temperature reached 50 °C, acrylonitrile (ACN), 32.43 g (0.61 mol), was pumped into the reactor over 2 h. When the addition was complete, the reaction mixture was cooled, and analyzed by gas chromatography (GC). The results are shown in Table 1.

### Examples 2-5

The procedure of Example 1 was repeated using the charge quantities and reaction temperatures shown in Table 1.

### Example 6

A 1.8 L Mettler-Toledo RC1 reactor was charged with 430 g (7.2 mol) EDA and 62 g (3.4 mol) H₂O. The agitator was started, the reactor was purged with nitrogen and then heated to 70 °C. When the reaction mixture reached the desired temperature, ACN, 759 g (14.3 mol), was pumped into the reactor over 4 h. The reaction mixture was stirred at 70 °C for 0.5 h after the ACN addition was completed. The reactor was cooled to ambient temperature, and the product was discharged. Analysis of the product by GC (water-free basis) in Table 1 showed that it contained 2.45% CNEDA, 90.31% BCNEDA, and 5.92% TCNEDA.

### Example 7

A 2 gallon Parr reactor was charged with 1522 g (25.3 mol) EDA and 211 g (11.7 mol) H₂O. The agitator was started, the reactor was purged with nitrogen and then heated to 70 °C. When the reaction mixture reached the desired temperature, ACN, 2688 g (50.6 mol), was pumped into the reactor over 4 h. The reactor was cooled to ambient temperature, and the product was discharged. Analysis of the product by GC (water-free basis) in Table 1 showed that it contained 4.63% CNEDA, 90.01% BCNEDA, and 5.02% TCNEDA.

### Example 8 (Comparison)

The method of Example 6 was repeated using 397.7 g (6.6 mol) EDA and 703.9 g (13.3 mol) ACN. No water was added to the reaction mixture. Analysis of the product by GC showed that it contained 12.25% CNEDA, 75.04% BCNEDA, and 12.30% TCNEDA on a water-free basis.

**Table 1**

| Ex | EDA g | ACN g | H₂O g/wt%* | °C | % CNEDA | % BCNEDA | % TCNEDA |
|---|---|---|---|---|---|---|---|
| 1 | 18.4 | 32.4 | 0/0 | 50 | 11.24 | 74.86 | 11.22 |
| 2 | 17.5 | 30.9 | 2.5/4.9 | 50 | 3.04 | 90.82 | 5.00 |
| 3 | 17.5 | 30.9 | 2.5/4.9 | 60 | 4.59 | 89.67 | 5.01 |
| 4 | 16.6 | 29.3 | 5.1/10 | 60 | 4.05 | 92.73 | 2.61 |
| 5 | 14.7 | 25.9 | 10.2/20 | 70 | 4.14 | 93.78 | 1.53 |
| | | | | | | | |
| 6 | 430 | 759 | 62/5 | 70 | 2.45 | 90.31 | 5.92 |
| 7 | 1522 | 2688 | 211/4.8 | 70 | 4.63 | 90.01 | 5.02 |
| 8 | 397.7 | 703.9 | 0/0 | 70 | 12.25 | 75.04 | 12.30 |

| | | | | | | | |
|---|---|---|---|---|---|---|---|
| Acrylonitrile added to EDA or EDA+H₂O over 2 hr for Exs 1-5 and over 4 hr for Exs 6-8. In each of Examples 1-8 the ACN:EDA molar ratio was 2.0. Analytical results are provided on a water-free basis. * % is based on total amount of EDA, ACN and water | | | | | | | |

Examples 1-8 show that the addition of water to the cyanoethylation reaction increases the selectivity to the dicyanoethylated product.

### Example 9

The equipment described in Example 6 was used. The reactor contained an initial charge of 250 g of nitrile mixture containing 90.9% BCNEDA, 1.1 % CNEDA, and 5.1 % TCNEDA on an anhydrous basis. This mixture contained about 5 wt% H₂O. The agitator was started, the reactor was purged with nitrogen then heated to 70 °C. EDA 379.1 g (6.3 mol) and ACN 670.4 g (12.6 mol) were pumped simultaneously from separate feed pumps over 4 hours. After the addition was completed, the mixture was cooled to ambient temperature. Analysis of the product by GC (water-free basis) in Table 2 showed that it contained 8.1% CNEDA, 68.8% BCNEDA, and 30.5% TCNEDA. The concentration of H₂O in the crude product at the end of the reaction was approximately 1 wt%. Subtracting the contributions from the heel, the composition would be approximately 10% CNEDA, 64% BCNEDA, and 24% TCNEDA on a water-free basis.

### Example 10

The product from the reaction above was charged as a heel (250 g), and the experiment was repeated under exactly the same conditions. As shown in Table 2, the product from this reaction contained 10.3% CNEDA, 57.8% BCNEDA, and 24.2% TCNEDA as determined by GC analysis on a water-free basis. The H₂O content was approximately 0.2 wt%.

### Example 11

The procedure of Example 9 was followed except that H₂O was added to the EDA charge. The reactor contained an initial charge of 250 g of nitrile mixture containing 90.9% BCNEDA, 1.1 % CNEDA, and 5.1 % TCNEDA on an anhydrous basis. This mixture contained about 5 wt% H₂O. The agitator was started, the reactor was purged with nitrogen then heated to 70 °C. Water, 52.6 g (2.9 mol), was mixed with 364.9 g EDA (total = 417.5 g; 6.1 mol EDA). This mixture was pumped into the reactor over 4 h. Simultaneously, 643.6 g ACN (12.1 mol) was pumped to the reactor over 4 h using a separate feed pump. After the addition was completed, the mixture was cooled to ambient temperature. Analysis of the product by GC (water-free basis) in Table 2 showed that the composition was 6.1 % CNEDA, 79.8% BCNEDA, and 12.1 % TCNEDA. Subtracting the contributions from the heel, the composition would be approximately 7% CNEDA, 77% BCNEDA, and 14% TCNEDA. The water content was 5 wt% of the total mixture.

### Example 12

The procedure of Example 9 was followed except that 125 g (6.9 mol) H₂O was charged to the reactor initially and there was no initial charge of a nitrile mixture. The agitator was started, the reactor was purged with nitrogen then heated to 62 °C. EDA 379.9 g (6.3 mol) and ACN 670.4 g (12.6 mol) were pumped simultaneously to the reactor from separate pumps over 4 h. The temperature increased to 70 °C within 0.5 h of the start of the additions, and was maintained at 70 °C for the remainder of the reaction. When the additions were complete, the reaction mixture was cooled, and the product was withdrawn and analyzed by GC. As shown in Table 2, the product contained 3.1 % CNEDA, 89.3% BCNEDA, and 6.4% TCNEDA (anhydrous basis). The water content was 10.6 wt% of the total mixture.

### Table 2.

**Table 2**

| Ex | EDA g | ACN g | H₂O g/wt%* | %/(%-heel) CNEDA | %/(%-heel) BCNEDA | %/(%-heel) TCNEDA |
|---|---|---|---|---|---|---|
| 9 | 379.1 | 670.4 | 12.5/1 | 8.1/10 | 68.8/64 | 30.5/24 |
| 10 | 379.1 | 670.4 | 2.5/0.2 | 10.3/10.9 | 57.8/55.3 | 24.2/25.1 |
| 11 | 364.9 | 643.6 | 65.1/5 | 6.1/7 | 79.8/77 | 12.1/14 |
| 12 | 379.9 | 670.4 | 125/10.6 | 3.1 | 89.3 | 6.4 |

In each of Examples 9-12 the ACN:EDA molar ratio was 2.0 and the reaction temperature was 70°C.

Examples 9-12 show that addition of water to the cyanoethylation reaction increases the selectivity to the dicyanoethylated product under an alternative mode of reaction in which EDA and ACN are added to the reactor simultaneously. The water may be charged to the reactor as a heel or it may be mixed with EDA.

### Hydrogenation of Cyanoethylated Ethylenediamine

Unless otherwise indicated, the composition of the cyanoethylated ethylenediamine mixture used for the hydrogenation Examples 13-17 was 6.83% CNEDA, 86.79% BCNEDA, and 6.00% TCNEDA.

### Example 13

### Batch Hvdroqenation in the Presence of NH3

A 1.8 L Mettler-Toledo RC1 reactor was used for this reaction. The reactor was charged with 1000 ml cyanoethylated ethylenediamine mixture and 15 g Raney® cobalt 2724 catalyst. The agitator was started at 1000 rpm, and the reactor was purged with nitrogen. Ammonia, 100 g, was pumped into the reactor. The reactor was pressurized with hydrogen to 27.5 bar, and the mixture was heated to 75 °C. After 4.5 h, the temperature was increased to 80 °C. The reactor was maintained under these conditions until hydrogen uptake ceased. The reaction mixture was cooled then the reactor was vented and purged with nitrogen. The product was withdrawn, filtered, and analyzed by GC. The product from this reaction contained 9.27% AEPD, 65.70% BAPED, and 3.56% TAPED on a solvent-free basis.

### Example 14

### Batch Hydrogenation over LiOH-modified Catalyst

The equipment described Example 13 was used for this reaction. The reactor was charged with 710 ml cyanoethylated ethylenediamine mixture, 8.9 g Raney® cobalt 2724 catalyst, and 0.7 g LiOH.H₂O in 6.9 g H₂O. The agitator was started, and the reactor was purged with nitrogen then with hydrogen. The reactor was pressurized with hydrogen to 27.5 bar then heated to 70 °C. After 335 L hydrogen had been consumed, the temperature was increased to 80 °C. The reactor was maintained under these conditions until hydrogen uptake ceased. The reaction mixture was cooled then the reactor was vented and purged with nitrogen. The product was withdrawn, filtered, and analyzed by GC. The product from this reaction contained 8.25% AEPD, 76.62% BAPED, and 4.93% TAPED on a water-free basis.

### Example 15

### Semi-batch Hydrogenation in Isopropanol Solvent

The cyanoethylated ethylenediamine product of Example 7 was hydrogenated in a 2 gallon Parr reactor. The reactor was charged with 600 g isopropanol and 60 g Raney® cobalt 2724 catalyst. The reactor was purged with nitrogen then with hydrogen. The agitator speed was set to 1000 rpm, and the reactor was heated under hydrogen pressure to 120 °C. When the temperature reached 120 °C, the pressure was increased to 800 psig. The cyanoethylated ethylenediamine product, 4400 g, was added to the mixture of catalyst and solvent over 4 h. At the end of the addition, the reaction mixture was cooled then the reactor was vented and purged with nitrogen. The product was discharged from the reactor and filtered. Analysis of the product by GC showed that it contained 3.71% AEPD, 87.19% BAPED, and 4.62% TAPED on a solvent-free basis.

### Example 16

### Semi-batch Hydrogenation in Isopropanol Solvent Using LiOH-Modified Catalyst

The reactor described in Example 13 was used for this reaction. The reactor was charged with 298 ml isopropanol, 10.07 g Raney® cobalt 2724 catalyst, and 10 g H₂O. A solution containing 2.52 g LiOH.H₂O (0.06 mol) in 22.83 g H₂O was added. The reactor was sealed and purged with nitrogen then hydrogen and pressurized with hydrogen to 35 bar. The reaction mixture was heated to 120 °C then the hydrogen pressure was increased to 55 bar. Cyanoethylated ethylenediamine containing 4.37% CNEDA, 88.92% BCNEDA, and 4.38% TCNEDA ,1004 g, was added to the hydrogenation reactor over 4 h. The reaction was terminated about 10 min after the addition was complete. The reactor was cooled, vented, and purged with nitrogen. The product was discharged through an internal filter at 70 °C. The catalyst and 297 g of the product were left in the reactor. Analysis of the product by GC showed that the composition was 4.16% AEPD, 87.28% BAPED, and 6.17% TAPED on a solvent-free basis.

### Example 17

### Semi-batch Hydrogenation Using Reaction Product as Heel and LiOH-modified Catalyst

The product from Example 16, 297 g, was used as the heel for this example. The same catalyst that was used in Example 16 also was used. The reactor was purged with nitrogen and hydrogen then was pressurized with hydrogen to 35 bar. The reaction mixture was heated to 120 °C then the reactor was pressurized to 55 bar with hydrogen. The cyanoethylated diamine used in Example 16, 999.8 g, was charged over 4 h. The reaction was terminated 5 min after the addition was complete. The reactor was cooled, vented, and purged with nitrogen. The product was discharged through an internal filter. A GC analysis of the product showed that the composition was 3.96% AEPD, 87.94% BAPED, and 5.97% TAPED on a solvent-free basis.

Examples 13-17 show various hydrogenation conditions suitable for hydrogenating the cyanoethylated EDA mixture.

## Claims

1. In a method for making N,N'-bis(2-cyanoethyl)ethylenediamine which comprises reacting acrylonitrile and ethylenediamine in a 1.6-2.4:1 molar ratio, the improvement for improving the selectivity of the reaction to N,N'-bis(2-cyanoethyl)-ethylenediamine which comprises reacting acrylonitrile and ethylenediamine in the presence of 2-30 wt% water, based on total reactants.

2. The method of Claim 1 in which the acrylonitrile and ethylenediamine molar ratio is 1.8-2.2:1.

3. The method of Claim 1 in which the acrylonitrile and ethylenediamine molar ratio is about 2:1.

4. The method of Claim 1 in which the acrylonitrile and ethylenediamine are added simultaneously and continuously to the reaction.

5. The method of Claim 1 in which the acrylonitrile is added continuously to the reaction.

6. The method of Claim 1 in which the reaction is run at a temperature from 20 to 90 °C.

7. The method of Claim 2 in which the water is present at 3-25 wt%.

8. The method of Claim 3 in which the water is present at 3-25 wt%.

9. The method of Claim 2 in which the water is present at 5-20 wt%.

10. The method of Claim 3 in which the water is present at 5-20 wt%.

11. In a method for making N,N'-bis(2-cyanoethyl)ethylenediamine which comprises reacting acrylonitrile and ethylenediamine in about a 2:1 molar ratio, the improvement for improving the selectivity of the reaction to N,N'-bis(2-cyanoethyl)-ethylenediamine which comprises reacting acrylonitrile and ethylenediamine in the presence of 5-20 wt% water, based on total reactants, the reaction temperature from 20 to 90 °C.

12. In a method for making N,N'-bis(3-aminopropyl)-1,2-ethylenediamine which comprises reacting acrylonitrile and ethylenediamine in a 1.6-2.4:1 molar ratio to make a N,N'-bis(cyanoethyl)-1,2-ethylenediamine reaction product and hydrogenating the reaction product, the improvement for improving the selectivity of the reactions to N,N'-bis(2-cyanoethyl)-ethylenediamine and to N,N'-bis(3-aminopropyl)-1,2-ethylenediamine which comprises reacting acrylonitrile and ethylenediamine in the presence of 2-30 wt% water, based on total reactants.

13. The method of Claim 12 in which the acrylonitrile and ethylenediamine molar ratio is about 2:1.

14. The method of Claim 12 in which the acrylonitrile and ethylenediamine are added simultaneously and continuously to the reaction.

15. The method of Claim 12 in which the acrylonitrile is added continuously to the reaction.

16. The method of Claim 12 in which the cyanoethylation reaction is run at a temperature from 20 to 90 °C.

17. The method of Claim 12 in which the hydrogenation reaction is run at a hydrogen pressure of 7 to 110 bar and a temperature from 70 to 150 °C.

18. The method of Claim 17 in which the hydrogenation reaction comprises a Raney metal catalyst.
